Europäisches Patentamt

European Patent Office

Office européen des brevets

(19)

(11) Publication number: **0 189 784**
**B1**

(12) **EUROPEAN PATENT SPECIFICATION**

(45) Date of publication of patent specification: **08.11.89**

(21) Application number: **86100482.8**

(22) Date of filing: **16.01.86**

(51) Int. Cl.⁴: **C 07 K 13/00, C 12 N 15/00,**
**C 12 N 9/99, C 07 H 21/04,**
**C 12 P 21/02, A 61 K 37/02,**
**A 61 K 37/64 // C12R1:19**

(54) **Human natural inhibitor of collagenases.**

(30) Priority: **18.01.85 US 692808**

(43) Date of publication of application:
**06.08.86 Bulletin 86/32**

(45) Publication of the grant of the patent:
**08.11.89 Bulletin 89/45**

(84) Designated Contracting States:
**BE CH DE FR GB IT LI NL SE**

(56) References cited:
**EP-A-0 103 409**

**JOURNAL OF THE NATIONAL CANCER INSTITUTE, vol. 69, no. 5, November 1982 (Washington DC); U.P. THORGEIRSSON et al. "Effect of Neutral Protease Inhibitors and a Chemoattractanct on Tumor Cell Invasion in Vitro" pages 1049-1054**

**THE BIOCHEMICAL JOURNAL, vol. 195, 1981, (London); G. MURPHY et al. "An inhibitor of collagenase from human amniotic fluid" pages 167-170**

(73) Proprietor: **G.D. Searle & Co.**
**P.O. Box 1045**
**Skokie Illinois 60076 (US)**

(72) Inventor: **Galloway, William Alan**
**77 Deeds Grove**
**High Wycombe (GB)**
Inventor: **Clissold, Patricia Mary**
**44 Arundel Road**
**Sands High Wycombe (GB)**
Inventor: **McCullagh, Keith Graham**
**30 Manor Park**
**Princes Risborough (GB)**

(74) Representative: **Wolff, Hans Joachim, Dr.jur. Dipl.-Chem. et al**
**Beil, Wolff & Beil Rechtsanwälte Postfach 80 01 40 Adelonstrasse 58**
**D-6230 Frankfurt am Main 80 (DE)**

(56) References cited:
**THE BIOCHEMICAL JOURNAL, vol. 218 (1984), pages 277-280**

**J. Biol. Chem. 258/20 (1983), 12252-12258**

Courier Press, Leamington Spa, England.

## Description

BACKGROUND OF THE INVENTION

(1) Field of the Invention

This invention describes the novel amino acid sequence of a human inhibitor of collagenases (NIC) and its production and use. Also described are novel DNA and RNA sequences, plasmids, transformed prokaryotic and eukaryotic cells, the production of NIC amino acid sequences with and without glycosylation, and the use of these novel NICs to treat diseases that incorporate collagenase-type metalloproteinases as part of the disease process.

(2) Description of the Prior Art

There is a need for the discovery of inhibitors of collagenase and related metalloproteinases and methods for their production and pharmaceutical use in the treatment of diseases in which connective tissue damage is caused by the metalloproteinases.

Connective tissues are maintained in dynamic equilibrium by the opposing effects of cellular connective tissue synthesis and extracellular matrix degradation. The extracellular matrix consists predominantly of collagens, with proteoglycans, fibronectin, laminin and other minor components making up the remainder.

Degradation of the matrix is brought about by the release of neutral metalloproteinases from resident connective tissue cells and invading inflammatory cells that are capable of degrading at physiological pH most of the matrix macromolecules (Murphy, G., Cawston, T. E., Galloway, W. A., Barnes, M. J., Bunning, R. A. D., Mercer, E., Reynolds, J. J., & Burgeson, R. E. (1981) Biochem. J. *199* 807—811). The proteinases include the mammalian tissue collagenases, proteoglycanase and gelatinases; leukocyte collagenase and gelatinase (Murphy, G., Reynolds, J. J., Bretz, U., & Baggiolini M., (1982) Biochem. J. *203* 209—221); macrophage collagenase and elastase (Werb, Z. & Gordon S. (1975) J. Exp. Med. *142* 346—360; and Banda, J. M., & Werb, Z., (1981) Biochem. J. *193* 589—605) and tumour collagenases (Liotta, L. A., Abe, S., Robey, P. G., & Martin, G. R. (1979) Proc.Natl.Acad.Sci. USA *76* 2268—2272; and Liotta, L. A., Lanzer, W. L., & Garbisa, S. (1981) Biochem. Biophys. Res. Commun. *98* 184—190). For a general review of collagenase and its role in normal and pathological connective tissues see "Collagenase in Normal and Pathological Connective Tissues", edited by David E. Woolley and John M. Evanson, John Wiley & Sons Ltd., 1980. The underlying basis of degradative diseases of connective tissue points to the matrix-specific metalloproteinases as having a fundamental role in the aetiology of these diseases. This is exemplified by Eisen for epidermolysis bullosa where the disease is linked to the overproduction of collagenase (Bauer, E. A., & Eisen, A. Z. (1978) J. Exp. Med. *148* 1378—1387. In rheumatoid arthritis, the work of Evanson and coworkers (Evanson, J. M., Jeffrey, J. J. & Krane, S. M. (1968) J. Clin. Invest. *47* 2639—2651) provided the discovery that large amounts of collagenase are produced, in culture, by excised rheumatoid synovial tissue. This led to immunolocalisation studies, by Woolley (Woolley, D. E., Crossley, M. J., Evanson, J. M. (1977) Arthritis & Rheu. *20* 1231—1239) with monospecific antibodies directed against human rheumatoid synovial collagenase which detected high levels of immunoreactive collagenase at the sites of joint erosion (cartilage-pannus junctions) but not in the cartilage or associated chondrocytes, and not in the synovium at sites remote from the resorbing front. Collagenase has also been implicated as the agent responsible for the degradation of collagen in corneal ulceration and, indeed, collagenase inhibitors are used in the treatment of this disorder (Brown, S. & Hook C., 1971 J. Ophthalmol. *72* 1139—1142; Slansky, Dohlman, Berman, 1970 *2* 488—491). In the field of tumor invasion the metastatic potential of some particular tumours correlates with the increased potential to synthesis and secrete collagenases (Liotta, L. A., Tryggvason, K., Garbisa, S., Hart, I., Foltz, C. M., Shafie, S. (1980) Nature *284*) or the inability to secrete significant amounts of (Hicks., N. J., Ward, R. V. & Reynolds, J. J. (1984) Int. J. Cancer, *33*, 835—844). Most of the studies mentioned above have limited the measurement of metalloproteinases to collagenase (the most widely studied of this group of metalloproteinases). However, it is known that cells and tissues secrete in addition to collagenase significant quantities of related metalloproteinases with additional matrix degrading specificities. It is therefore understood that the simultaneous effects of several metalloproteinases will exacerbate the degradation of the connective tissue over that achieved by collagenase alone.

Thus it is perceived that specific inhibitors of collagenase-like metalloproteinases when used in pharmacological formulation are likely to modify the pathogenesis and provide a beneficial therapy for diseases of connective tissues characterised by matrix degradation of which the following are examples: rheumatoid arthritis; corneal, epidermal or gastric ulceration; emphysema; dystrophic epidermolysis bullosa; tumour metastasis or invasion; periodontal disease; and bone disease. Also, the use of such agents is likely to be useful in the treatment of any disorder where excessive matrix loss is caused by metalloproteinase activity and in the promotion of wound healing following surgery.

NIC, isolated from connective tissue fibroblasts, is a potent inhibitor of mammalian collagenases. NIC binds to collagenase with 1:1 stoichiometry and with high affinity such that the inhibition of collagenase by NIC is virtually irreversible (Cawston, T. E., Murphy, G., Mercer, E., Galloway, W. A., Hazleman B. L., Reynolds, J. J. (1983) Biochem. J. *211* 313—318). The high affinity means that physiologically NIC will be able to compete successfully against collagen (Km for human rheumatoid synovial collagenase is 2 ×

$10^{-6}$M) even when the collagen is in considerable molar excess.

We assert that therapeutic administration of human NIC produced by the methods of this invention is genetically engineered microorganisms or mammalian cells is useful in the clinical treatment of connective tissue disorders. Such genetic engineering methods provide sufficient material to enable the clinical testing of NIC and unglycosylated NIC as a demonstration of its safety and efficacy.

Specific natural inhibitors of collagenase were discovered in crude medium from cultured connective tissues. Since their discovery they have been systematically studied and biochemically characterised although mainly in relation to their physicochemical properties and the biochemistry of their interaction with collagenase (Cawston, T. E., Murphy, G., Mercer, E. (Galloway, W. A., Hazleman, B. L., Reynolds, J. J. (1983) Biochem. J. *211* 313—318; and Welgus, H. G., Stricklin, G. P., Eisen, A. Z., Bauer, E. A., Cooney, R. V., Jeffrey, J. J. (1978) J.Biol.Chem. *254* 1938—1943). Human NIC is a glycosylated single chain polypeptide with approximately 12% carbohydrate. The first twenty-three amino terminal amino acids of a hyman fibroblast inhibitor have been elucidated and described by Stricklin, G. P., & Welgus, H. G. (1983) J.Biol.Chem. *258* 12252—12258 together with its amino acid composition. The composition of the material isolated by Stricklin and Welgus has significant differences when compared to the composition of the matter disclosed in this Patent, most strikingly Stricklin and Welgus claim that their inhibitor contains 24½-cysteine residues whereas the matter claimed in this patent contains 12½-cysteine residues. Similarly, other amino acids of Stricklin and Welgus differ in their composition. Also the unambiguous identification of residues 8 and 15 of the first 23 amino acids was not disclosed by Stricklin and Welgus. The composition of the carbohydrate and details of its linkages to the protein are not known, except that the carbohydrate has specificity that allows it to interact with concanavalin A-sepharose (Murphy, G., Cawston, T. E., Reynolds, J. J. (1981) Biochem. J. *195* 167—170) a property that aids purification of the inhibitor.

Thus the structure of the mature (full length) fibroblast NIC is not known nor has the structure of the DNA coding for natural NIC been elucidated. It follows that the mechanisms by which NIC inhibits collagenase is also unknown. Biological testing "in vivo" of natural fibroblast derived NIC has not been disclosed. In an "in vitro" cell culture model of tumour cell migration through a natural basement membrane, rabbit bone culture media derived NIC was able to arrest the migration of a collagenase secreting tumour cell line through such a natural membrane (Thorgeirsson, U. P., Liotta, L. A., Kalebic, T. Margulies, I. M., Thomas, K., Rios-Cadelore, M. (1982) JNCl, *69* 1049—1054.

BRIEF DESCRIPTION OF THE DRAWINGS

Figure 1 shows the entire sequence of the cDNA clone NIC 37—1, as determined by DNA sequence analysis.

Figure 2 shows the nucleotide and amino acid sequence of human NIC. The amino acid sequence was derived from the DNA sequence and is shown above the DNA sequence. Amino acids are numbered from the first residue of the mature protein through to residue 184. The signal sequence is numbered negatively. Potential glycosylation sites are underlined beneath the amino acid sequence. The polyadenylation signal is uderlined beneath the nucleic acid sequence.

Figure 3 shows the structure of the cDNA clone NIC 37—1 in relation to the encoded NIC protein. Some restriction sites are marked. The open box represents untranslated regions, the cross-hatched box the signal sequence and the filled-in box the mature NIC protein.

Figure 4 shows the general formula of all the possible synthetic cDNA sequences that can code for human NIC. The amino acid sequence is shown together with the possible DNA codon sequences theoretically able to code for each amino acid.

Figure 5 shows the sequence of 37 of the first 39 N terminal amino acids of the natural fibroblast derived purified NIC protein.

Figure 6 shows a yeast shuttle vector pAYE4.

Figure 7 shows a yeast shuttle vector pAYE4 with a yeast DNA sequence inserted into the HINDIII site to form pAYE4(34). The insert contains the pyruvate kinase gene and 5' and 3' flanking yeast DNA.

Figure 8 shows the sequence of the synthetic oligonucleotide comprising the AvaII to HindIII pyruvate kinase promoter fragment.

Figure 9 shows the construction of pAYE85 which reconstitutes the pyruvate kinase promoter by incorporating the synthetic oligonucleotide.

Figure 10 shows the construction of pAYE86 which contains the yeast pyruvate kinase promoter followed by the alcohol dehydrogenase terminator. This plasmid has two HINdIII restriction endonuclease sites, one of which separates the promoter and terminator.

Figure 11 shows the removal of the unwanted HindIII restriction endonuclease site to yield pAYE89. This expression plasmid permits the insertion of gene inserts at the Hind III site separating promoter and terminator.

DEFINITIONS

NIC refers to natural inhibitors of collagenase (and metalloproteinases related to collagenase) that have the amino acid sequence defined in this patent. They include inhibitors (of metalloproteinases) synthesised by fibroblasts, chondrocytes, endothelial cells, tumor cells and connective tissue cells.

Collagenase refers to enzymes capable of degrading collagens and matrix macromolecules.

Proteoglycanases refers to enzymes capable of degrading proteoglycanase and other matrix macromolecules. Gelatinases refers to enzymes capable of degrading gelatin and other matrix macromolecules.

SUMMARY OF THE INVENTION

The present invention is directed to the means and methods of producing human NIC via recombinant DNA technology including, (1) the discovery of the amino acid sequence of residues 1—49 allowing the design of specific oligonucleotide probes; (2) the discovery and identity of the entire DNA sequence of the mature protein as well as its signal polypeptide, the 3' and 5' flanking region thereof and therefore the complete 184 amino acid sequence of the mature NIC protein; (3) the construction of cloning and expression vehicles comprising said DNA sequence, coding for the human NIC protein, as well as [Met,] fusion or signal N-terminus conjugates thereof; and (4) viable cell cultures, genetically altered by producing human NIC in physical state distinct from its existence in, or isolation from, a natural environment or source, such NIC, by virtue of its method of preparation described herein, being essentially free of human endogenous proteins and other native materials and substances.

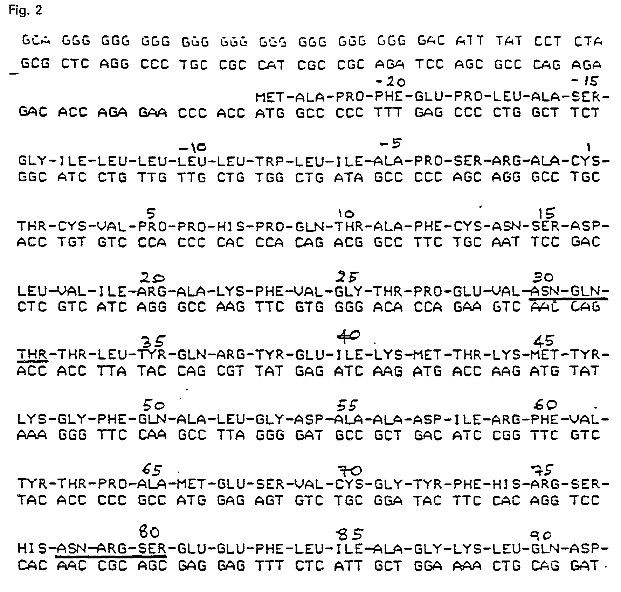

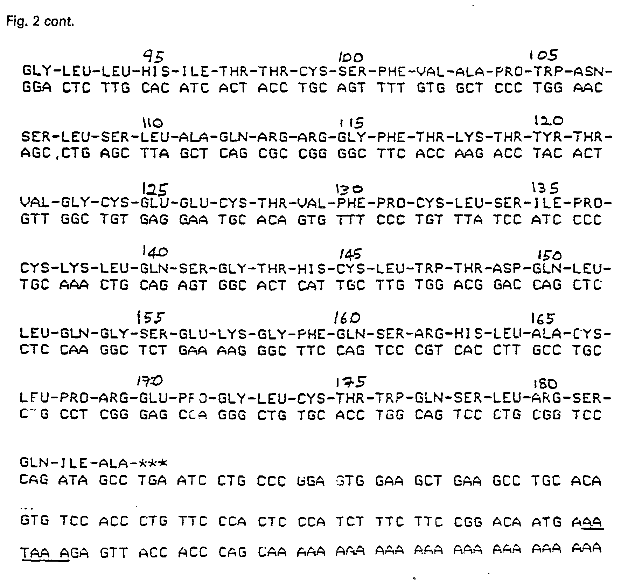

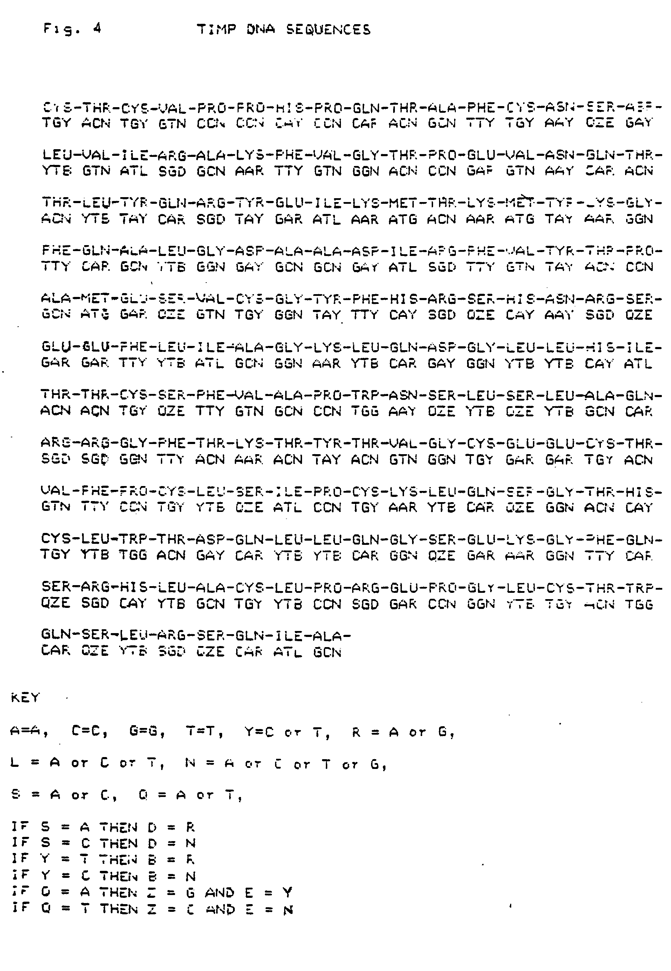

The NIC sequence may be accompanised by natural mammalian glycosylation, by yeast glycosylation or without glycosylation. A DNA or RNA sequence coding for the synthesis of NIC may be expressed in a plasmid in a eukaryotic or prokaryotic cell and is described by the polynucleotide sequence in Figure 2. One object of the invention is the production of natural mammalian NIC in mammalian cells through the use of an extra-chromosomal element or the addition of multiple copies of NIC gene into the cellular chromosome. The DNA sequences coding for NIC are described in Figure 4.

Similarly, RNA polynucleotides can be utilised to synthesise NIC. The RNA sequence is the same as the DNA sequence with deoxyribose replacing ribose and uracil replacing thymidine.

A plasmid containing an autonomously replicating element in combination with a DNA polynucleotide coding for NIC can be used in prokaryotic or eukaryotic systems. The prokaryotes include bacteria, such as E.coli. The eukaryotics include yeast and vertebrate cells such as mammalian or avian. The plasmids coding for NIC are utilised to transfect the cells, the cells are grown under conditions which promote the production of NIC and the NIC is isolated from both the cells and the media. The NIC is utilised to inhibit metalloproteinases by bringing NIC into contact with them. The contact can be achieved using a pharmaceutical composition suitable for use by injection, oral, topical, aerosol, or suppository administration.

The metalloproteinases include but are not limited to collagenases, proteoglycanases, gelatinases, elastases, leukocyte metalloproteinases, tumour metalloproteinases, macrophage metalloproteinases and connective tissue cell metalloproteinases.

The pharmaceutical composition may contain NIC at a concentration of at least 1.0 nanogram per milliliter. In a more preferred embodiment the NIC concentration is at least 1.0 milligram per milliliter of pharmaceutical composition. The pharmaceutical composition is administered in a pharmaceutically effective amount for the treatment of disease conditions where metalloproteinases are active in degrading macromolecules such as in connective tissues. Among the many diseases where NIC pharmaceutical compositions are useful as rheumatoid arthritis; corneal, epidermal or gastric ulceration; peridontal disease states; tumours or invasive cancer; dystrophic epidermolysis bullosa; and emphysema. The administration of the NIC compositions may be systemic or local, it may be accomplished by injection, topical, oral, aerosol or suppository application.

The invention is directed to the recombinant DNA production of human NIC in all of its aspects, and is not to be construed as limited to any specific details described herein and embraced within the compass of this invention.

DETAILED DESCRIPTION OF THE PREFERRED EMBODIMENTS

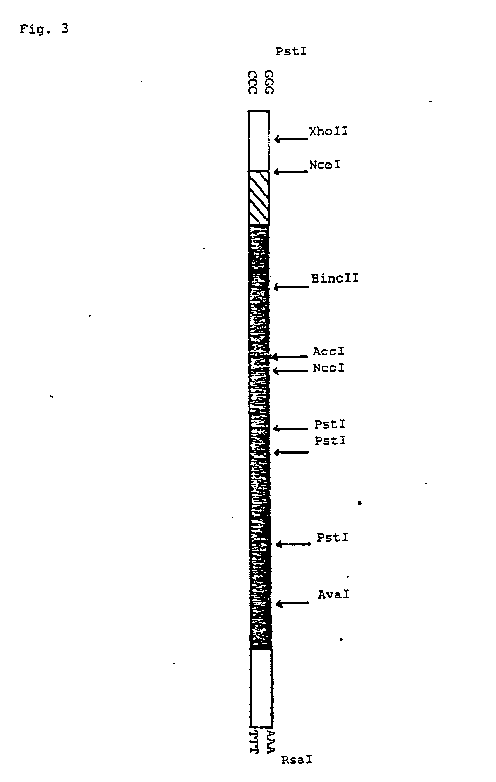

The NIC amino acid sequence was determined by first isolating the natural NIC molecule and determining the sequence of 47 of the initial 49 amino acids from the N-terminus. From this amino acid sequence a mixture of 24 synthetic DNA sequences were synthesised which was known to contain one sequence exactly complementary to the mRNA coding for NIC. The poly-A mRNA was isolated from human fibroblast cells and cDNA was prepared using reverse-transcriptase, and used to transform E.coli. The mixture of 24 synthetic DNA sequences were hybridized to the plasmid DNA on replica plates and 2 positive colonies containing plasmid NIC cDNA were detected and evaluated. The cDNA coding for NIC was sequenced and confirmed to be the NIC DNA sequence by comparison with the known 49 amino acid sequence of natural NIC. The comparison confirmed that the cDNA sequence isolated was the sequence coding for NIC. The NIC amino acid sequence is illustrated in Figure 2. Also shown in Figure 2 is the cDNA sequence and the amino acid sequence of the natural human leader sequence. The restriction pattern of the cDNA is shown in Figure 3. The DNA polynucleotides that will code for the amino acid sequence are illustrated in Figure 4. The NIC cDNA coding sequences were excised from the cloning vector at the 5' XhoII site and the RsaI at the 3' end. This DNA sequence coding for NIC was placed between the unique HindIII and BamHI sites of yeast shuttle-vector pAYE89. DHI E.coli cells transformed with this construct are ATCC number 39945. The construct without NIC DNA sequences inserted (pAYE89) in DHI cells is ATCC 39944.

The yeast cell used for expression of NIC protein with shuttle-vector pTC306 is strain X4003—5B (Berkeley Yeast Stock Center, California, ATCC 20791). The expressed NIC protein produced by vector pTC306-transformed yeast cells was assayed by testing *in vitro* and was found to be active as an inhibitor of metallocolagenase.

## Example 1
### Identification of first 49 amino acids

(1) Cell Culture

Primary human foreskin fibroblast were cultured in roller bottles and grown up in RPMI 1640 (Gibco) supplemented with 10% calf serum. On reaching confluence the cells were changed to Dulbecco's Modified Eagles Medium (serum free) and maintained in this medium prior to harvesting the medium for NIC isolation.

(2) Protein Purification and Analysis

The harvested medium was concentrated 100x, using a Millipore Pellicon Apparatus fitted with a 10,000 M.W. cut-off PTGC cassette, and stored at $-20°C$ till required. Collagenase activity was assayed by measuring the degradation of $^{14}C$ labelled collagen using the assay described by Cawston, T. E. and Barrett, A. J. 1979 Anal.Biochem. *399* 340—345. Inhibitor activity was assayed by determining the amount of sample required to inhibit 50% of the activity of a known amount of collagenase in the standard assay. NIC was purified from the concentrated medium using heparin-Sepharose chromatography, DEAE-Sepharose chromatography and Concanavalin A-Sepharose chromatography as described by conditions described in Cawston, T. E., Galloway, W. A., Mercer, E., Murphy, G., and Reynolds, J. J. 1981 Biochem. J. *195* 159—165. This provided protein which was honogeneous by SDS-gel electroporesis of $^{125}I$ labelled purified NIC. To obtain 5 mg of protein approximately 200 L of culture medium was processed.

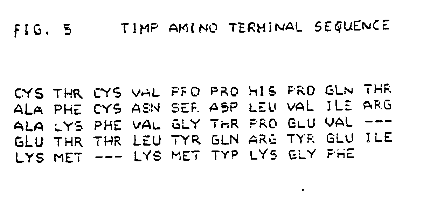

To obtain protein sequence data, 0.5 mg of the purified NIC was de-salted on Sepharose LH60 equilibrated with 30% formic acid in methanol. Protein sequence analysis was carried out under contract by Applied Biosystems Incorporated using a Gas Phase Microsequenator. This analysis resulted in the identification of 47 of the first 49 amino terminal acid residues (Figures 5).

## Example 2
### Isolation of a plasmid containing the NIC gene

(1) Messenger RNA Isolation

Primary human foreskin fibroblasts were cultured to confluency in RMPI-1640 supplemented with 10% calf serum. Cells were detached from the surface by trypsinisation and were washed in PBS and pelleted. Total RNA from the fibroblasts was extracted by the method of Penman et al (Penman, S., Smith, I., Waltzman (1966) Science *154* 786). DNA was removed from the RNA by extraction with 3M sodium acetate as described by Parish and Kirby (Parish, J. H., Kirby, K. (1966) Biochem. Biophys. Acta *129* 554). Poly A mRNA was purified from total RNA by oligo-dT cellulose chromatography (Ario, H. and Leder, P. (1972) Proc. Natl. Acad. Sci. USA *69* 1408—1412).

(2) Preparation and Cloning of Complementary DNA to Fibroblast Cell Messenger RNA

Okayama and Berg vectors were prepared by the published procedure Okayama, H., & Berg, P., (1982) Mol.Cell.Biol. *2* 161—170. Poly A mRNA from the fibroblasts was annealed to the Okayama and Berg dT tailed vector under the conditions described by these authors. Approximately 2 pmoles of mRNA was incubated under annealing conditions with 1 pmole of dT tailed vector. The mRNA was reverse-transcribed into cDNA using the dT tails of the vector as an oligo dT primer for the reverse-transcriptase. The reaction mix was standard except that RNasin was present to inhibit mRNA degradation. The cDNA was dC tailed, the vector circularised, the cDNA made double stranded, and the dG-dC and dT-dA joint repaired, all as described by Okayama and Berg.

The ligation mix containing vector-cDNA molecules was used to transform E. coli K12 strain DH 1 to ampicillin resistance. The cells were made competent by the method of Hanahan, D., (1983) J.Mol.Biol. *166* 557—580. The efficiency of transformation obtained was $5 \times 10^7$ colonies per microgram of pBR322 closed covalent circular DNA. About 25,000 recombinant colonies were obtained from lul of 36—4 mRNA.

(3) Preparation of Synthetic DNA Oligomers for use as NIC Screening Probes

A mixture of 24 synthetic DNA oligomers, each 15 bases long were synthesised by the phosphoramidate method. These were designed to all possibilities from the section of mRNA encoding amino acids 38 to 42 inclusive of the NIC protein:

| Amino Acid — Number from N terminus | 38 | 39 | 40 | 41 | 42 |
|---|---|---|---|---|---|
| — Identification | Tyr — | Glu — | Ile — | Lys — | Met |
| Possible Codons | T | A | T | A | |
| | TA | GA | ATC | AA | ATG |
| | C | G | A | G | |

**(4) Screening the Library**

The transformed DHI cells were immediately spread onto LM agar plates containing 100 µg/ml of ampicillin. Ater 15 hours growth at 37°C, colonies were replica plated onto nitrocellulose (S&S BA85) filters. The filters were placed on LM agar plates containing 100 µg/ml of ampicillin to maintain the replica colonies. The master-plates were re-incubated at 37°C to regenerate the original colonies. The replica colony filters were transferred to LM agar plates containing 100 µg/ml of ampicillin and 100 µg/ml of chloramphenicol and replica colonies were incubated at 37°C overnight to amplify their plasmids. The DNA from each colony was then denatured and fixed to the filter by the procedure of Grunstein, M., & Hogness, D., (1975) Proc.Natl.Acad.Sci. 72 3961. Filters were prehybridized for four hours at 60°C in 4×SET, (1×SET = 0.15M NaCl, .03M Tris-Cl, 2 mM EDTA, pH 7.4) 10 × Denhardt's, 100 µg/ml polyrA, 100 µg/ml polyrC, 50 µg/ml denatured E. coli DNA, 0.1% sodium pyroposphate and 0.1% sodium lauryl sulphate. The solution was then changed to a fresh solution of the above containing 10 pmole/ml of the radiolabelled oligonucleotide probe mixture, specific activity $5 \times 10^6$ cpm/pmole. Hybridization was at 30°C for 15 hours. Filters were washed extensively in 4×SET at 30°C, followed by one wash in 1×SET at 30°C and finally by a 5 minute wash in 1×SET at 37°C. Filters were exposed to Kodak XR—5 X-ray film with DuPont Lightning-Plus intensifying screens for 2 hours at −70°C.

**(5) Characterization of Plasmid DNA**

4 colonies hybridized with the mixture of 24 synthetic oligomeric DNA probes. Plasmid DNA was isolated from these colonies by a rapid miniscreen method (Holmes, D. S. & Quigley, M., (1981). Anal.Biochem. 114 193) and the DNA was double-digested with PstI and PvuII restriction enzymes. 2 plasmids had inserts of just less than 1Kbs, one plasmid had an insert of 4 Kb and one an insert of 2.2 Kbs. A large batch of CsCl purified plasmid DNA was prepared from each of these bacterial colonies.

The DNAs were extensively restriction mapped and Southern Blots of Pst I—Pvu II digests of each plasmid DNA were made. The Southern Blots were hybridized to the mixture of 24 synthetic oligomeric DNA probes at 30°C in 4×SET as described above. Blots were then washed at 37°C in 1×SET and exposed to X-ray film with intensifying screens at −70°C for 3 hours. All the plasmid cDNA inserts hybridized with the probe mixture. The blots were then washed at 47°C in 1×SET and re-exposed to X-ray film as described, but for 24 hours. The 2.2 Kb cDNA insert no longer hybridized to the probes, and so was eliminated.

Of the two plasmids containing cDNA inserts of less than one kilobase, one insert was estimated as 960 bases and the other as 860 bases. Restriction mapping showed that the 860 base insert was a 5' truncated version of the 960 base cDNA insert.

Therefore the 960 base cDNA clone, NIC 37—1, was preferred for sequencing. To define whether the remaining clone, NIC 6—1, containing a 4Kb insert, or the NIC 37—1 clones containing the 960/860 base inserts, encoded the NIC protein, a second synthetic DNA probe was designed. This probe was 24 bases long and utilized the preferred mammalian codons encoding the amino acids 35 to 42 of NIC protein:—

| [35]tyr | gln | arg | tyr | glu | ile | lys | met[42] | |
|---------|-----|-----|-----|-----|-----|-----|---------|---|
| UAC | CAG | AGG | UAC | GAG | AUC | AAG | AUG | Preferred mRNA codons |
| 3'ATG | GTC | TCC | ATG | CTC | TAG | TTC | TAC | 5' ANTISENSE PROBE |

The probe was the antisense strand complementary to the mRNA and was not a mixture as before, as only the preferred mammalian codon for each amino acid was synthesized.

The NIC 37—1 (960 b) and NIC 6—1 (4 kb) cDNA inserts were cut out of their vectors by Hind III—Pvu II restriction digestion. Each of the two cDNA inserts were AluI—Hae III digested and the resulting fragments were cloned into bacteriophage M13 mp8, cut with Sma I, and dephosphorylated. The recombinant M13 bacteriophage were amplified from plaques and single-stranded recombinant M13 DNA was prepared by standard techniques (M13 Cloning and Sequencing Handbook (1983) Publ. by Amersham Int.). The single-stranded DNAs were dot-blotted onto nitrocullulose filters, and were hybridized to the single 24 base oligomeric DNA probe, in 4×SET at 35°C. DNA from two out of 60 recombinant NIC 37—1 M13 phage hybridized but none of the DNA prepared from 166 recombinant NIC 6—1 M13 phage hybridized. The original plasmids were denatured and dot-blotted also. DNA from NIC 6—1 cDNA clone did not hybridize whereas DNA from NIC 37—1 did hybridize to the single 24 base synthetic DNA probe. Thus the longer, single probe differentiated between the two clones whereas the mixture of 24 oligomers, each 15 bases long, could not. An M13 NIC 37—1 clone which hybridized to the 24 oligomeric probe was sequenced by the dideoxy method. One of the 3 reading frames of the sequence obtained corresponded to the part of the NIC protein whose amino acid sequence was known, and thus clone NIC 37—1 was confirmed to encode the NIC protein.

## Example 3

Amino acid sequence of NIC

(1) Sequencing

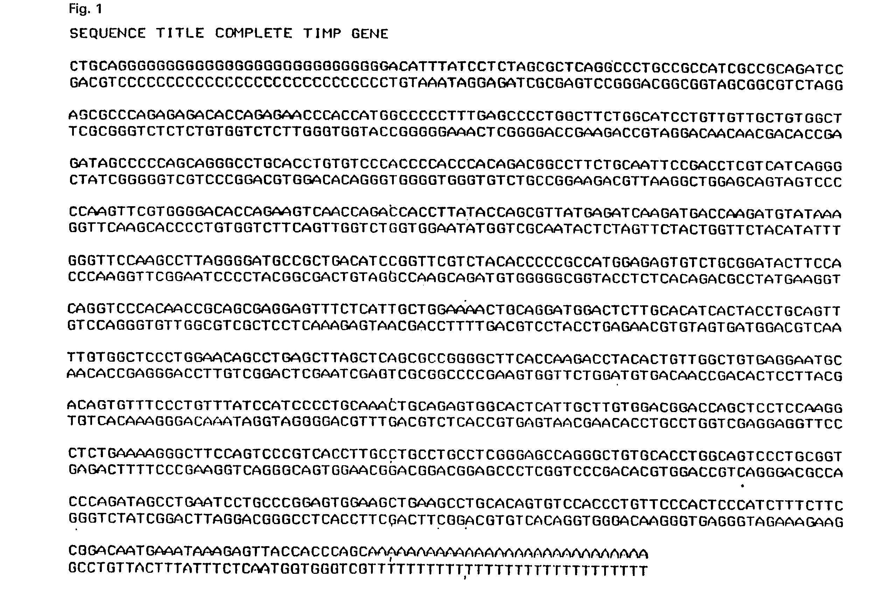

The cDNA insert of NIC clone 37—1 was sequenced by the Maxam and Gilbert procedure (Maxam, A., and Gilbert, W., (1980) Methods in Enzymol. 65, 499) and by dideoxy sequencing after subcloning fragments into the M13 vector mp 8 (Messign, J. & Vieira, J., (1982) *19* 269—276). The full sequence is shown in Figure 1. Clone 37—1 was found to contain a sequence 798 base-pairs in length (the dG.dC tail of 29 bases at the 5'' end and the dA-dT tail of some 120 bases at the 3' end of the insert are not included). There is an open reading frame of 621 nucleotides encoding a protein of 207 amino acids initiated with the amino acid methionine (Figure 2). The sequence around the ATG initiation codon concords with the consensus sequence $^A_C$AXXATGG for eukaryotic initiation reported by Kozak ((Kozak, M., (1981) Nucleic Acids Res. *9* 5233—5252).

The 5' untranslated region is 77 bases long and the 3' untranslated region is 100 bases long. The polyadenylation signal AATAAA precedes the first dA of the 3' tail by 15 residues. The first 23 amino acids of the protein derived from the DNA sequence correspond to a typical signal sequence of an exported mammalian protein. These amino acids are labelled −1 to −23 in Figure 2. The amino acid marked +1 on Figure 2 is the same as the N-terminus amino acid of the secreted NIC isolated from the human foreskin fibroblast medium, which would be expected if the signal sequence is cleaved off during secretion. Amino acids +1 to +49 derived from the DNA sequence of clone 37—1 coincide with the N-terminus amino acid sequence obtained by peptide sequencing of secreted NIC protein. There are two possible glycosylation sites AsnX$^{Thr}_{Ser}$, which are underlined in Figure 2.

Figure 3 shows the structure of the cDNA insert of 37—1 with some restriction sites referenced with respect to the positions of the non-coding (open box) and coding (solid box) regions of the nucleic acid sequence. The signal sequence within the coding region is cross-hatched in the diagram.

## Example 4

### Synthesis of DNA coding for NIC

Based upon the amino acid sequence of NIC disclosed in Figure 2 other DNA sequences can be constructed which also code for the synthesis of NIC. The potential DNA sequences coding for NIC are described in Figure 4. The following table describes the DNA sequences which could code for each amino acid.

## TABLE

| Amino Acid | DNA | | Amino Acid | DNA |
|---|---|---|---|---|
| Arg | SGD | | Gly | GGN |
| Leu | YTB | | Val | GTN |
| Ser | QZE | | Lys | AAR |
| Thr | ACN | | Asn | AAY |
| Pro | CCN | | Gln | CAR |
| Ala | GCN | | His | CAY |
| Glu | GAR | | Asp | GAY |
| Tyr | TAY | | Cys | TGY |
| Phe | TTY | | Ile | ATL |
| Met | ATG | | Trp | TGG |

KEY:

A = A,  C = C,  G = G,  T = T,  Y = C or T,  R = A or G,

L = A or C or T,  N = A or C or T or G,

S = A or C,  Q = A or T,

IF S = A THEN D = R
IF S = C THEN D = N
IF Y = T THEN B = R
IF Y = C THEN B = N
IF Q = A THEN Z = G AND E = Y
IF Q = T THEN Z = C AND E = N

The individual DNA sequences can be constructed using standard polynucleotide synthetic procedures such as the phosphoramidate method. Similarly RNA sequences can be constructed where the sequence is the same as that of the DNA in Figure 4, but where ribose replaces deoxyribose and uracil replaces thymidine.

The DNA sequences in Figure 4 coding for NIC can be inserted into prokaryotic and eukaryotic cells. Insertion for transformation is usually accomplished by plasmids but can also be accomplished using transforming viruses. The NIC DNA can also be inserted into the host cell DNA to increase the gene dosage allowing increased NIC synthesis.

Plasmids containing the NIC gene can be expressed in bacterial cells to produce NIC without glycosylation. Plasmids in yeast or vertebrate cells can be expressed to produce NIC with the corresponding yeast or vertebrate glycosylation. In mammalian cells, such as human cells, the NIC produced is identical in character to that secreted from normal human fibroblasts.

Example 5
Construction of a yeast expression vector

The pyruvate kinase gene together with its 5' and 3' flanking sequences including the pyruvate kinase promoter and terminator was isolated by standard techniques (Burke et al. 1983). A yeast shuttle vector pAYE 89 was constructed that was able to replicate in both yeast and *E.coli*. The plasmid permits a foreign gene sequence to be inserted and permits the expression of that gene under the control of the pyruvate kinase promoter.

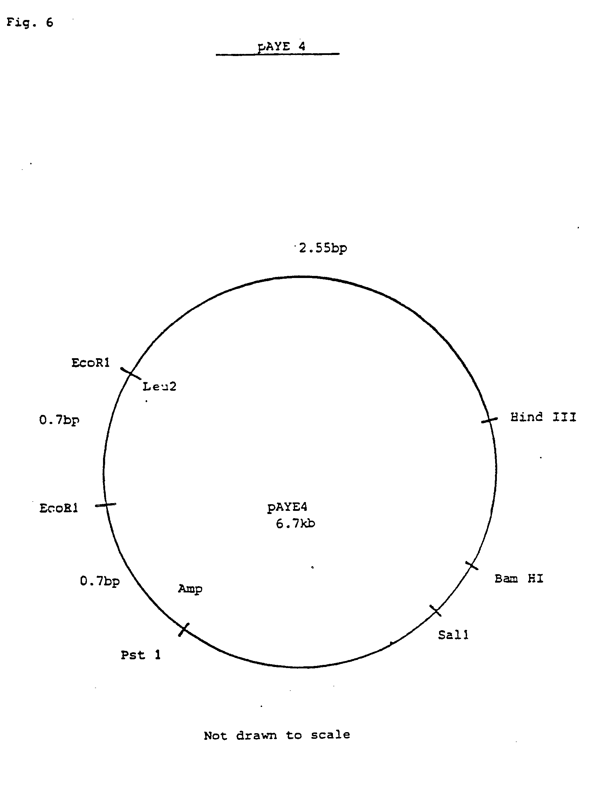

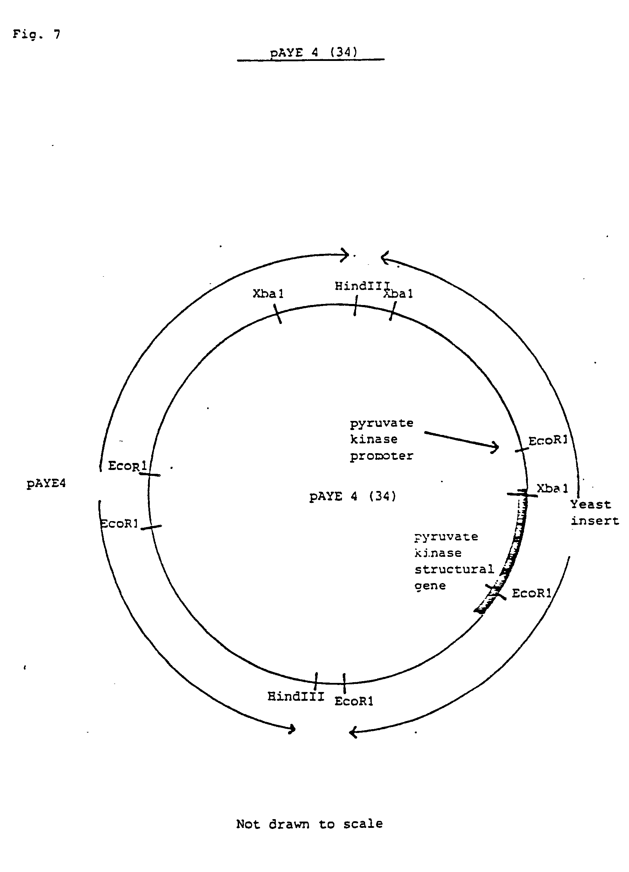

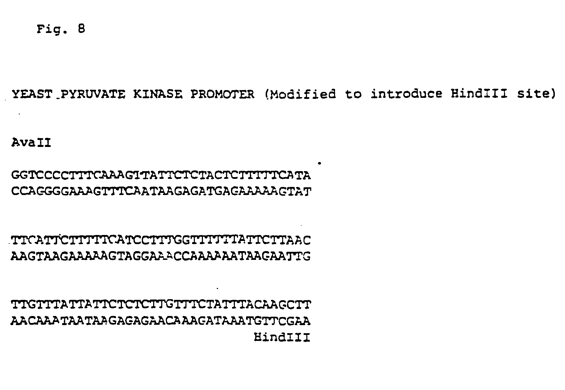

A Hind III DNA restriction fragment (c. 5bp) was inserted into the Hind III site of plasmid pAYE 4 (Figure 6) to produce pAYE 4 (34) (Figure 7). This plasmid was digested to completion with the restriction endonucleases EcoRI and XbaI to produce eight DNA fragments. The c.470 bp fragment was isolated. This fragment which spans the promoter and the structural gene contains an Ava II restriction endonuclease site. The fragment was cut into two further fragments of 330 bp and 140 bp in length by incubation with Ava II restriction endonuclease and the 330 bp fragment was isolated. The EcoRI—AvaII fragment was ligated to the synthetic oligonucleotide shown in Figure 8 and a 440 bp Eco RI to Hind III fragment containing a single Ava II restriction site isolated.

This EcoRI—Hind III fragment was inserted into pBR322 after digestion of the plasmid wit EcoRI and Hind III restriction endonucleases and recovery of the 4207 bp fragment.

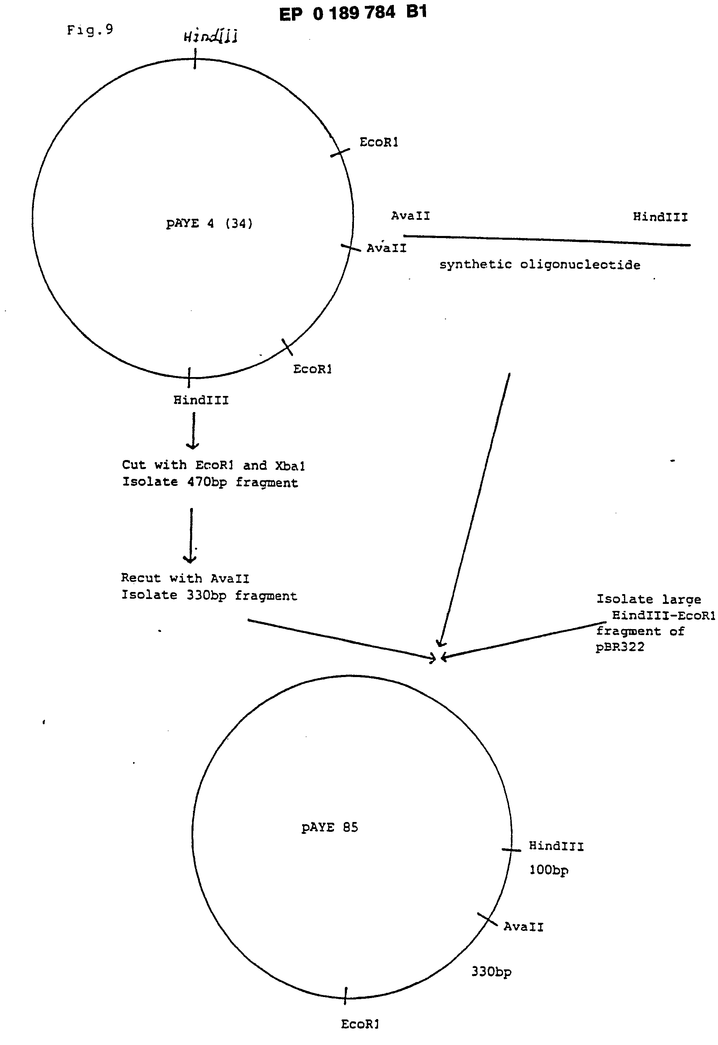

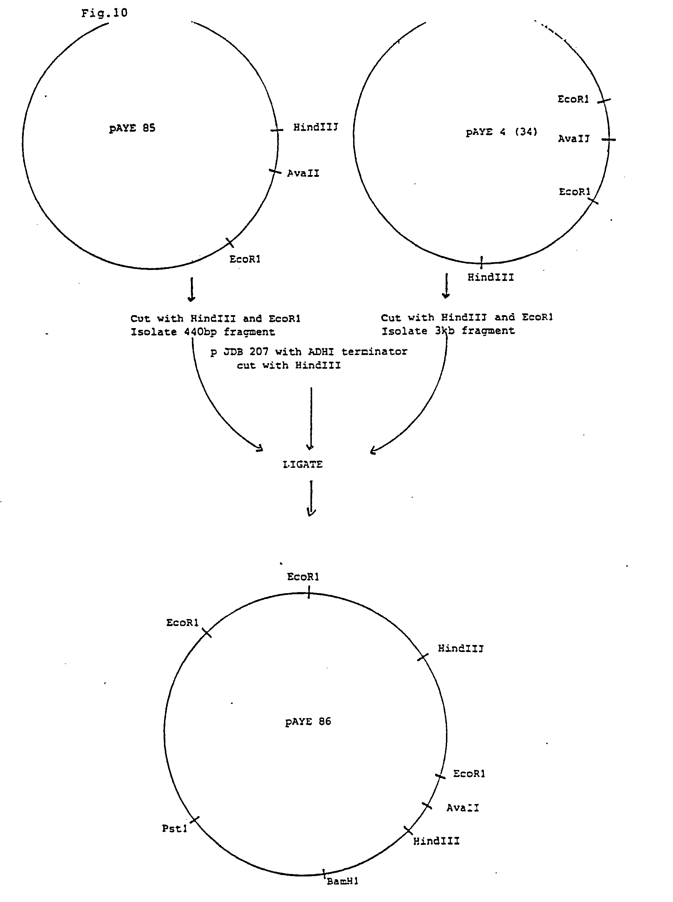

The resulting plasmid pAYE(85) (Figure 9) was cut with Hind III and EcoRI restriction endonucleases and the c. 440 bp EcoRI to Hind III DNA fragment isolated. pAYE 4 (34) was cut with the same two enzymes and a 3 kb Hind III—EcoRI fragment was isolated. pjDB207 containing the ADHI terminator was cut with Hind III restriction endonuclease. The three fragments containing upstream promoter sequences, downstream promoter sequences and ADHI terminator sequences were ligated together to give pAYE 86 (Figure 10).

This plasmid has the pyruvate kinase promoter and alcohol dehydrogenase terminator I (ADHI) separated by a Hind III restriction endonuclease site. There is a further Hind III restriction site upstream from the pyruvate kinase promoter. This latter site was eliminated after partial digestion of the plasmid with Hind III restriction endonuclease to linearise the plasmid.

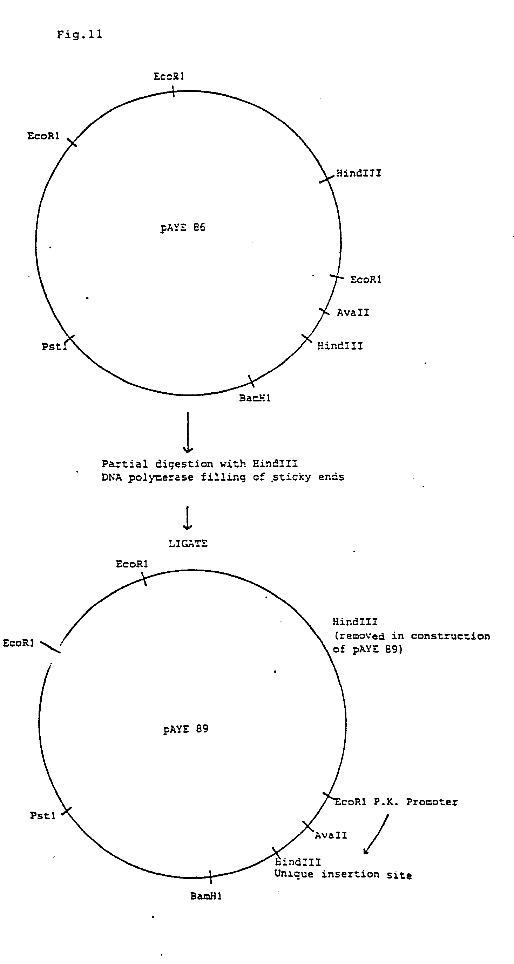

The resulting plasmid, pAYE 89, has a unique Hind III restriction site between the pyruvate kinase promoter and the alcohol dehydrogenase I terminator that permits the insertion of any foreign structural gene (Figure 11).

## (1) Construction of a yeast expression vector for NIC

The complete NIC gene, including the signal sequence, the 3'-untranslated region and 35 bp from the 5'-untranslated region, was inserted into the yeast shuttle plasmid, pAYE89, such that expression is under control of the PK (pyruvate kinase) promoter.

### (a) Preparation of vector

Plasmid pAYE 89 (10 µgs) was digested with HindIII (30 units) in 150 µl of 50 mM NaCl, 50 mM Tris HCl pH 7.8 and 10 mM MgCl$_2$ at 37°C for 2 hours. The reaction was then made 150 mM NaCl and incubated at 37°C for a further 3 hours with Bam HI (25 units). Digestion was terminated by incubating the reaction at 65°C for 10 minutes followed by extraction with phenol. DNA was recovered by ethanol precipitation, washed with 80% ethanol, dried and resuspended in 25 µl of 10 mM Tris pH 7.8. The resulting sticky ends were rendered blunt by incubation with 5 units of DNA polymerase I (Klenow fragment) in 50 µl of 50 mM Tris HCl pH 7.8, 10 mM MgSO$_4$, 0.1 mM DTT, 200 µM dATP, 200 µM dCTP; 200 µM dGTP and 200 µM dTTP for 30 minutes at 30°C. Following incubation at 65°C for 10 minutes to inactivate the polymerase, the large vector fragment (about 8100 bps) was isolated by gel electrophoresis on a 1% agarose gel in TBE buffer. The fragment was recovered by electroelution onto DE52 followed by ethanol precipitation. After an 80% Ethanol wash the dried pellet was resuspended in 200 µls of 50 mM Tris-HCl pH 7.8, 10 mM MgCl$_2$ and incubated at 37°C for 1 hour with 20 units of Calf Intestinal Phosphatase to remove 5'-terminal phosphate groups. Protein was removed by phenol extraction and the DNA recovered by Ethanol precipitation washed and dried. It was then resuspended in 30 µl of 10 mM Tris-HCl pH 7.5, 1 mM EDTA at a concentration of 50 µg/ml.

### (b) Preparation of NIC gene insert

Plasmid T37—1 (40 µgs) was digested with endonuclease Rsa I (50 units) in 300 µls of 50 nM Tris-HCl pH 7.8, 50 mM NaCl and 10 mM MgClz for 4 hours at 37°C. Following the addition of 100 µls of gel loading buffer the reaction was heated at 65°C for 10 minutes and then fractionated on a preparative 1.2% agarose gel in TBE buffer. The second largest band (980 bps), containing the entire NIC gene was electroeluted onto DE52 and recovered by ethanol precipitation. The DNA pellet was washed with 80% ethanol, dried and resuspended in 300 µls of 10 mM Tris-HCl pH 8 and 10mM MgO$_2$. It was then digested with 30 units of XhoII for 4 hours at 37°C to produce a 926 bp fragment containing 35 bases of the 5'-untranslated region, the entire NIC coding region and the 3'-untranslated region with the poly A tail. This was then purified on a 1.2% agarose gel in TBE buffer and recovered by electroelution onto DE52 and ethanol precipitation. The dried pellet was dissolved in 25 µls of 10 mM Tris-HCl pH 7.5, 1 mM EDTA. The XhoII sticky end was made blunt using 5 units of DNA polymerase I (Klenow fragment) in 60 µls of 50 mM Tris-HCl pH 7.8, 10 mM MgSO$_4$, 0.1 mM DTT, 400 µM dATP, 400 µM dCTP, 400 µM dGTP and 400 µM dTTP at 30°C for 30 minutes. Following extraction with phenol the fragment was precipitated with ethanol. The DNA pellet was washed with 80% ethanol, dried and resuspended in 30 µls of 10 mM Tris-HCl pH 7.5 and 1 mM EDTA.

### (c) Ligation and Transformation

Blunt ended vector and gene fragments at a molar ration of 2:1 were then ligated together by T4 DNA ligase (400 units, BIOLABS) in 100 µls of 50 mM Tris-HCl pH 8, 10 mM MgCl$_2$, 20 mM DTT and 1 mM ATP for 16 hours at 15°C. This ligation mix was then used to transform E.coli K12 strain BJ/l to ampicillin resistance by the standard procedure. Plasmids containing the NIC gene in the correct orientation were identified by restriction analysis and used to transform E.coli K12 strain HB101. DNA sequence analysis on plasmid DNA purified from E.coli K12 strain HB101 transformants was used to confirm that the plasmid sequence was as designed.

(2) Expression of NIC in yeast

The plasmid pTC306 was introduced into yeast strain X4003—5B (Berkeley Yeast Stock Centre) by standard yeast transformation techniques (Beggs J. D. Nature *275* 104—109 (1978) and transformants selected on the basis of their ability to grow on complete medium minus leucine with 1.2 m sorbitol.

Individual clones were isolated by streaking onto complete medium minus leucine. Individual colonies were used to inoculate 10 ml. cultures of YEPD medium (1% yeast extract, 2% Bactopeptone, 2% glucose). Cultures were grown at 30°C to an $A_{660}$ of 1—2.

Cells were collected by centrifugation and the medium was assayed for NIC activity. Cells were resuspended in 1 ml. of phosphate buffered saline (20 mM sodium phosphate pH 7.0 plus 0.14 m NaCl) and 2 gr. of glass beads (0.45—50 mm diam). The cells were broken by vortexing 5 × 1 min with cooling in ice between vortex cycles. Cell extract was recovered and the cell debris removed by centrifugation. The cell-free extract was assayed for NIC biological activity, as described in Example 1. Amounts of NIC expressed varied from 0.1 to 0.8 units/ml (units are defined by Cawston, T. E. Murphy, G., Mercer, E., Galloway, W. A., Hazleman B. L., Reynolds, J. J. (1983) Biochem. J. *211* 313—318).

### Example 6
### Pharmaceutical Administration

The NIC molecule binds to metalloproteinases on a 1:1 basis which is essentially irreversible. We determined the NIC dissociation constant (Kd) to be $5 \times 10^{-11}$ molar. To be pharmaceutically effective the administration of NIC should be in an amount that effectively inhibits the metalloproteinases in the area to be treated. The NIC administration may be by any effective route including injection, intravenous, intramuscular or subcutaneous; topical in ointment or lotion; orally in a pharmaceutical composition that releases the NIC in the area to be treated; aerosol into the area to be treated; or suppository form that releases the NIC at a pharmaceutically effective rate.

The pharmaceutical compositions may be combined with other ingredients that are effective in the treatment of disease conditions wherein metalloproteinases are active. Among the preferred other ingredients are anti-inflammatory compounds and inhibitors of elastase (serine proteinase). These combinations with NIC are expected to better inhibit the complete range of cellular proteinase activities.

When applied, the composition may be combined with other ingredients, such as carriers. There are no limitations on the nature of such carriers except that they must be pharmaceutically acceptable. The composition may be ointments or suspensions, transdermal patches, plasters, bandages, pills, tablets, lozenges, suppositories or injectable liquids.

The NIC compositions may be used for parenteral administration along with acceptable carriers such as saline. The injection may be by any acceptable route including intramuscularly, intra-articularly, intravenously, or subcutaneously.

The amount of NIC in the pharmaceutical composition is an effective amount for treating the disease condition. The dosage is minimally the amount that is effective at inhibiting the metalloproteinase in the area to be treated. Expected dosages are to be with a pharmaceutical composition containing more than 1 nanogram per milliliter, preferably more than 1 milligram per milliliter of composition. Dosages administered are to be increased until inhibition of the metalloproteinases is complete. Further suitable carriers, compositions and formulations acceptable for pharmaceutical use are described in Remmington's Pharmaceutical Sciences by E. W. Martin which is hereby incorporated by reference.

Notwithstanding that reference has been made to particular preferred embodiments, it will be understood that the present invention is not to be construed as limited to such, rather to the lawful scope of the appended claims.

**Claims**

1. A composition of matter comprising the 184 Amino Acid Sequence of NIC of the formula

CYS-

THR-CYS-VAL-PRO-PRO-HIS-PRO-GLN-THR-ALA-PHE-CYS-ASN-SER-ASP-

LEU-VAL-ILE-ARG-ALA-LYS-PHE-VAL-GLY-THR-PRO-GLU-VAL-ASN-GLN-

THR-THR-LEU-TYR-GLN-ARG-TYR-GLU-ILE-LYS-MET-THR-LYS-MET-TYR-

LYS-GLY-PHE-GLN-ALA-LEU-GLY-ASP-ALA-ALA-ASP-ILE-ARG-PHE-VAL-

TYR-THR-PRO-ALA-MET-GLU-SER-VAL-CYS-GLY-TYR-PHE-HIS-ARG-SER-

HIS-ASN-ARG-SER-GLU-GLU-PHE-LEU-ILE-ALA-GLY-LYS-LEU-GLN-ASP-

GLY-LEU-LEU-HIS-ILE-THR-THR-CYS-SER-PHE-VAL-ALA-PRO-TRP-ASN-

SER-LEU-SER-LEU-ALA-GLN-ARG-ARG-GLY-PHE-THR-LYS-THR-TYR-THR-

VAL-GLY-CYS-GLU-GLU-CYS-THR-VAL-PHE-PRO-CYS-LEU-SER-ILE-PRO-

CYS-LYS-LEU-GLN-SER-GLY-THR-HIS-CYS-LEU-TRP-THR-ASP-GLN-LEU-

LEU-GLN-GLY-SER-GLU-LYS-GLY-PHE-GLN-SER-ARG-HIS-LEU-ALA-CYS-

LEU-PRO-ARG-GLU-PRO-GLY-LEU-CYS-THR-TRP-GLN-SER-LEU-ARG-SER-

GLN-ILE-ALA.

2. The composition of matter of Claim 1 in substantially pure form and accompanied by yeast glycosylation.

3. The composition of matter of Claim 1 in combination with 23 Amino Acid signal sequence.

4. The composition of matter of Claim 3 when accompanied by yeast glycosylation.

5. A composition of matter comprising a DNA polynucleotide in substantially pure form coding for the polypeptide NIC selected from the DNA structure of Figure 4.

6. The DNA polynucleotide of Claim 5 in substantially pure form comprising the sequence of Figure 2.

7. A DNA polynucleotide of Claim 6 in combination with a DNA polynucleotide coding for a signal sequence comprising the DNA sequence of Figure 2.

8. A composition of matter comprising an RNA polynucleotide with the polynucleotide sequence of Claim 5 wherein the deoxyribonucleotides are replaced by ribonucleotides and thymine is replaced by uracil.

9. A plasmid comprising an autonomously replicating element containing a DNA polynucleotide of Claim 5.

10. A plasmid of Claim 9 comprising the plasmid pTC306 ATCC 39945.

11. A method for the production of the compound of Claim 1 or Claim 2 comprising:
a) transforming a micro-organism with the plasmid of Claim 9;
b) fermenting the said transformed micro-organism;
c) isolating the compound of Claim 1 or Claim 2 from the fermentation.

12. The method of Claim 11 wherein the micro-organism is a prokaryotic cell.

13. The method of Claim 12 wherein the prokaryotic cell is Escherichia coli.

14. The method of Claim 11 wherein the micro-organism is a eukaryotic cell.

15. The method of Claim 14 wherein the eukaryotic cells is yeast.

16. The method of Claim 15 wherein the yeast is X4003—5B, ATCC 20791.

17. A method for the production of NIC comprising:
a) Transforming a vertebrate cell with the plasmid of claim 9;
b) Growing the transformed cell and producing NIC;
c) Isolating the NIC from the transformed cell and media.

18. The method of claim 17 wherein the vertebrate cell is a mammalian cell.

19. A method of inhibiting the action of any metallo-proteinase comprising the contacting of the metallo-proteinase and the compound of Claim 1 or Claim 2.

20. The method of Claim 19 wherein the metallo-proteinase is any collagenase.

21. The method of Claim 19 wherein the metallo-proteinase is any proteoglycanase.

22. The method of Claim 19 wherein the metallo-proteinase is any gelatinase.

23. The method of Claim 19 wherein the metallo-proteinase is any leukocyte metalloproteinase.

24. The method of Claim 19 wherein the metallo-proteinase is any macrophase metalloproteinase.

25. The method of Claim 19 wherein the metallo-proteinase is any tumour cell metalloproteinase.

26. A composition of matter for the treatment of any metalloproteinase dependent diseases

**EP 0 189 784 B1**

comprising a therapeutically effective amount of the compound of Claim 1 or Claim 2 in combination with a pharmaceutically acceptable carrier.

27. The composition of matter of Claim 26, wherein the compounds are present in an amount of at least 1.0 nanogram per milliliter of composition.

28. The composition of Claim 26, wherein the compounds are present in an amount of at least 1 milligram per milliliter of composition.

29. The composition of Claim 26, wherein the carrier is suitable for topical application.

30. The composition of Claim 26, wherein the carrier is suitable for injection.

31. The composition of Claim 26, wherein the carrier is suitable for an aerosol.

32. The composition of Claim 26, wherein the carrier is suitable for a suppository.

**Patentansprüche**

1. Eine Zusammensetzung, enthaltend die 184-Aminosäuresequenz der humanen natürlichen Collagenase-Inhibitoren (NIC) der Formel

CYS-

THR-CYS-VAL-PRO-PRO-HIS-PRO-GLN-THR-ALA-PHE-CYS-ASN-SER-ASP-

LEU-VAL-ILE-ARG-ALA-LYS-PHE-VAL-GLY-THR-PRO-GLU-VAL-ASN-GLN-

THR-THR-LEU-TYR-GLN-ARG-TYR-GLU-ILE-LYS-MET-THR-LYS-MET-TYR-

LYS-GLY-PHE-GLN-ALA-LEU-GLY-ASP-ALA-ALA-ASP-ILE-ARG-PHE-VAL-

TYR-THR-PRO-ALA-MET-GLU-SER-VAL-CYS-GLY-TYR-PHE-HIS-ARG-SER-

HIS-ASN-ARG-SER-GLU-GLU-PHE-LEU-ILE-ALA-GLY-LYS-LEU-GLN-ASP-

GLY-LEU-LEU-HIS-ILE-THR-THR-CYS-SER-PHE-VAL-ALA-PRO-TRP-ASN-

SER-LEU-SER-LEU-ALA-GLN-ARG-ARG-GLY-PHE-THR-LYS-THR-TYR-THR-

VAL-GLY-CYS-GLU-GLU-CYS-THR-VAL-PHE-PRO-CYS-LEU-SER-ILE-PRO-

CYS-LYS-LEU-GLN-SER-GLY-THR-HIS-CYS-LEU-TRP-THR-ASP-GLN-LEU-

LEU-GLN-GLY-SER-GLU-LYS-GLY-PHE-GLN-SER-ARG-HIS-LEU-ALA-CYS-

LEU-PRO-ARG-GLU-PRO-GLY-LEU-CYS-THR-TRP-GLN-SER-LEU-ARG-SER-

GLN-ILE-ALA.

2. Die Zusammensetzung gemäss Anspruch 1 in im wesentlichen reiner Form und begleitet von Hefe-Glycosylierung.

3. Die Zusammensetzung gemäss Anspruch 1 in Kombination mit der 23-Aminosäuresignalsequenz.

4. Die Zusammensetzung gemäss Anspruch 3 begleitet von Hefe-Glycosylierung.

5. Eine Zusammensetzung, enthaltend ein DNA-Polynucleotid in im wesentlichen reiner Form, welches für das NIC-Polypeptid codiert, ausgewählt aus der DNA-Struktur gemäss Figur 4.

6. Das DNA-Polynucleotid gemäss Anspruch 5 in im wesentlichen reiner Form, enthaltend die Sequenz gemäss Figur 2.

7. Ein DNA-Polynucleotid gemäss Anspruch 6 in Kombination mit einem DNA-Polynucleotid, welches eine Signalsequenz codiert, enthaltend die DNA-Sequenz gemäss Figur 2.

8. Eine Zusammensetzung, enthaltend ein RNA-Polynucleotid mit der Polynucleotid-Sequenz gemäss Anspruch 5, worin die Desoxyribonucleotide durch Ribonucleotide ersetzt sind und Thymin durch Uracil ersetzt ist.

9. Ein Plasmid, enthaltend ein autonom replizierendes Element, enthaltend ein DNA-Polynucleotid gemäs Anspruch 5.

10. Ein Plasmid gemäss Anspruch 9, enthaltend das Plasmid pTC306 mit der ATCC-Hinterlegungsnummer 39945.

11. Ein Verfahren zur Herstellung der Verbindung gemäss Anspruch 1 oder 2 umfassend:

a) einen Mikroorganismus mit dem Plasmid gemäss Anspruch 9 transformieren,

b) diesen transformierten Mikroorganismus fermentieren,

c) die Verbindung gemäss Anspruch 1 oder 2 aus der Fermentation isolieren.

12. Das Verfahren gemäss Anspruch 11, worin der Mikroorganismus eine prokaryotische Zelle ist.

13. Das Verfahren gemäss Anspruch 12, worin die prokaryotische Zelle Escherichia coli ist.

14. Das Verfahren gemäss Anspruch 11, worin der Mikroorganismus eine eukaryotische Zelle ist.

15. Das Verfahren gemäss Anspruch 14, worin die eukaryotische Zelle Hefe ist.

16. Das Verfahren gemäss Anspruch 15, worin die Hefe diejenige mit der Bezeichnung X4003—5B der ATCC-Hinterlegungsnummer 20791 ist.

17. Ein Verfahren zur Herstellung von humanen natürlichen Collagenaseinhibitoren (NIC) umfassend:

a) eine Vertebrat-Zelle mit einem Plasmid gemäss Anspruch 9 transformieren,

b) die transformierte Zelle vermehren und NIC produzieren,

c) den NIC aus der transformierten Zelle und dem Medium isolieren.

18. Das Verfahren gemäss Anspruch 17, worin die Vertebrat-Zelle eine Säugetier-Zelle ist.

19. Ein Verfahren zur Inhibierung der Wirkung von irgendeiner Metallo-Proteinase, umfassend das Kontaktieren der Metallo-Proteinase und eine Verbindung gemäss Anspruch 1 oder 2.

20. Das Verfahren gemäss Anspruch 19, worin die Metallo-Proteinase irgendeine Collagenase ist.

21. Das Verfahren gemäss Anspruch 19, worin die Metallo-Proteinase irgendeine Proteoglycanase ist.

22. Das Verfahren gemäss Anspruch 19, worin die Metallo-Proteinase irgendeine Gelatinase ist.

23. Das Verfahren gemäss Anspruch 19, worin die Metallo-Proteinase irgendeine Leukocyten-Metalloproteinase ist.

24. Das Verfahren gemäss Anspruch 19, worin die Metallo-Proteinase irgendeine makrophage Metalloproteinase ist.

25. Das Verfahren gemäss Anspruch 19, worin die Metallo-Proteinase irgendeine Tumor-Zellen-Metalloproteinase ist.

26. Eine Zusammensetzung zur Behandlung von irgendeiner Metallo-Proteinase-abhängigen Erkrankung, enthaltend eine therapeutisch wirksame Menge einer Verbindung gemäss Anspruch 1 oder Anspruch 2 in Kombination mit einem pharmazeutisch zulässigen Träger.

27. Eine Zusammensetzung gemäss Anspruch 26, worin die Verbindungen in einer Menge von wenigstens 1,0 Nanogramm pro Milliliter der Zusammensetzung vorhanden sind.

28. Die Zusammensetzung gemäss Anspruch 26, worin die Verbindungen in einer Menge von wenigstens 1 Milligramm pro Milliliter Zusammensetzung vorhanden sind.

29. Die Zusammensetzung gemäss Anspruch 26, worin de Träger für die topische Anwendung geeignet ist.

30. Die Zusammensetzung gemäss Anspruch 26, worin de Träger für die Injektion geeignet ist.

31. Die Zusammensetzung gemäss Anspruch 26, worin de Träger für ein Aerosol geeignet ist.

32. Die Zusammensetzung gemäss Anspruch 26, worin de Träger für ein Suppositorium geeignet ist.

# EP 0 189 784 B1

**Revendications**

1. Composition comprenant la séquence de 184 amino-acides de l'INC de formule

CYS-

THR-CYS-VAL-PRO-PRO-HIS-PRO-GLN-THR-ALA-PHE-CYS-ASN-SER-ASP-

LEU-VAL-ILE-ARG-ALA-LYS-PHE-VAL-GLY-THR-PRO-GLU-VAL-ASN-GLN-

THR-THR-LEU-TYR-GLN-ARG-TYR-GLU-ILE-LYS-MET-THR-LYS-MET-TYR-

LYS-GLY-PHE-GLN-ALA-LEU-GLY-ASP-ALA-ALA-ASP-ILE-ARG-PHE-VAL-

TYR-THR-PRO-ALA-MET-GLU-SER-VAL-CYS-GLY-TYR-PHE-HIS-ARG-SER-

HIS-ASN-ARG-SER-GLU-GLU-PHE-LEU-ILE-ALA-GLY-LYS-LEU-GLN-ASP-

GLY-LEU-LEU-HIS-ILE-THR-THR-CYS-SER-PHE-VAL-ALA-PRO-TRP-ASN-

SER-LEU-SER-LEU-ALA-GLN-ARG-ARG-GLY-PHE-THR-LYS-THR-TYR-THR-

VAL-GLY-CYS-GLU-GLU-CYS-THR-VAL-PHE-PRO-CYS-LEU-SER-ILE-PRO-

CYS-LYS-LEU-GLN-SER-GLY-THR-HIS-CYS-LEU-TRP-THR-ASP-GLN-LEU-

LEU-GLN-GLY-SER-GLU-LYS-GLY-PHE-GLN-SER-ARG-HIS-LEU-ALA-CYS-

LEU-PRO-ARG-GLU-PRO-GLY-LEU-CYS-THR-TRP-GLN-SER-LEU-ARG-SER-

GLN-ILE-ALA.

2. Composition selon la revendication 1 sous une forme essentiellement pure et accompagnée d'une glycosylation de levure.

3. Composition selon la revendication 1 en combinaison avec la séquence-signal de 23 amino-acides.

4. Composition selon la revendication 3 accompagnée d'une glycosylation de levure.

5. Composition comprenant un polynucléotide de type ADN sous une forme essentiellement pure codant pour le polypeptide INC choisi parmi une structure d'ADN de la figure 4.

6. Polynucléotide de type ADN selon la revendication 5 sous une forme essentiellement pure comprenant la séquence de la figure 2.

7. Polynucléotide de type ADN de la revendication 6 en combinaison avec un polynucléotide de type ADN codant pour une séquence-signal comprenant la séquence d'ADN de la figure 2.

8. Composition comprenant un polynucléotide de type ARN avec la séquence polynucléotidique de la revendication 5, où les désoxyribonucléotides sont remplacés par des ribonucléotides et la thymine est remplacée par l'uracile.

14

9. Plasmide comprenant un élément responsable de la réplication autonome contenant un polynucléotide de type ADN de la revendication 5.

10. Plasmide selon la revendication 9 comprenant le plasmide pTC306 ATCC 39945.

11. Procédé sour la production du composé selon la revendication 1 ou de la revendication 2 comprenant:

a) la transformation d'un microorganisme avec le plasmide de la revendication 9;

b) une fermentation avec ledit microorganisme transformé;

c) l'isolement du composé de la revendication 1 ou de la revendication 2 à partir de la fermentation.

12. Procédé selon la revendication 11, dans lequel le microorganisme est une cellule procaryote.

13. Procédé selon la revendication 12, dans lequel la cellule procaryote est Escherichia coli.

14. Procédé selon la revendication 11, dans lequel le microorganisme est une cellule eucaryote.

15. Procédé selon la revendication 14, dans lequel la cellule eucaryote est une levure.

16. Procédé selon la revendication 15, dans lequel la levure est X4003—5B, ATCC 20791.

17. Procédé pour la production d'INC comprenant:

a) la transformation d'une cellule de vertébré avec le plasmide de la revendication 9;

b) la culture de la cellule transformée et la production d'INC;

c) l'isolement de l'INC à partir de la cellule transformée et du milieu.

18. Procédé selon la revendication 17, dans lequel la cellule de vertébré est une cellule de mammifère.

19. Procédé d'inhibition de l'action d'une métalloprotéinase quelconque comprenant le contact de la métalloprotéinase et du composé de la revendication 1 ou de la revendication 2.

20. Procédé selon la revendication 19, dans lequel la métalloprotéinase est une collagénase quelconque.

21. Procédé selon la revendication 19, dans lequel la métalloprotéinase est une protéoglycannase quelconque.

22. Procédé selon la revendication 19, dans lequel la métalloprotéinase est une gélatinase quelconque.

23. Procédé selon la revendication 19, dans lequel la métalloprotéinase est une métalloprotéinase leucocytaire quelconque.

24. Procédé selon la revendication 19, dans lequel la métalloprotéinase est une métalloprotéinase de macrophage quelconque.

25. Procédé selon la revendication 19, dans lequel la métalloprotéinase est une métalloprotéinase de cellule tumorale quelconque.

26. Composition pour le traitement des maladies dépendantes d'une métalloprotéinase quelconque comprenant une quantité thérapeutique efficace du composé de la revendication 1 ou de la revendication 2 en combinaison avec un véhicule pharmaceutiquement acceptable.

27. Composition selon la revendication 26, dans laquelle les composés sont présents en une quantité d'au moins 1,0 ng/ml de composition.

28. Composition selon la revendication 26, dans laquelle les composés sont présents en une quantité d'au moins 1 mg/ml de composition.

29. Composition selon la revendication 26, dans laquelle le véhicule convient à l'application locale.

30. Composition selon la revendication 26, dans laquelle le véhicule convient à l'injection.

31. Composition selon la revendication 26, dans laquelle le véhicule convient pour un aérosol.

32. Composition selon la revendication 26, dans laquelle le véhicule convient pour un suppositoire.

15

Fig. 1

SEQUENCE TITLE COMPLETE TIMP GENE

CTGCAGGGGGGGGGGGGGGGGGGGGGGGGGGGGGACATTTATCCTCTAGCGCTCAGGCCCTGCCGCCATCGCCGCAGATCC
GACGTCCCCCCCCCCCCCCCCCCCCCCCCCCCCCCCTGTAAATAGGAGATCGCGAGTCCGGGACGGCGGTAGCGGCGTCTAGG

AGCGCCCAGAGAGACACCAGAGAACCCACCATGGCCCCCTTTGAGCCCCTGGCTTCTGGCATCCTGTTGTTGCTGTGGCT
TCGCGGGTCTCTCTGTGGTCTCTTGGGTGGTACCGGGGGAAACTCGGGGACCGAAGACCGTAGGACAACAACGACACCGA

GATAGCCCCCAGCAGGGCCTGCACCTGTGTCCCACCCCACCCACAGACGGCCTTCTGCAATTCCGACCTCGTCATCAGGG
CTATCGGGGGTCGTCCCGGACGTGGACACAGGGTGGGGTGGGTGTCTGCCGGAAGACGTTAAGGCTGGAGCAGTAGTCCC

CCAAGTTCGTGGGGACACCAGAAGTCAACCAGACCACCTTATACCAGCGTTATGAGATCAAGATGACCAAGATGTATAAA
GGTTCAAGCACCCCTGTGGTCTTCAGTTGGTCTGGTGGAATATGGTCGCAATACTCTAGTTCTACTGGTTCTACATATTT

GGGTTCCAAGCCTTAGGGGATGCCGCTGACATCCGGTTCGTCTACACCCCCGCCATGGAGAGTGTCTGCGGATACTTCCA
CCCAAGGTTCGGAATCCCCTACGGCGACTGTAGGCCAAGCAGATGTGGGGGCGGTACCTCTCACAGACGCCTATGAAGGT

CAGGTCCCACAACCGCAGCGAGGAGTTTCTCATTGCTGGAAAACTGCAGGATGGACTCTTGCACATCACTACCTGCAGTT
GTCCAGGGTGTTGGCGTCGCTCCTCAAAGAGTAACGACCTTTTGACGTCCTACCTGAGAACGTGTAGTGATGGACGTCAA

TTGTGGCTCCCTGGAACAGCCTGAGCTTAGCTCAGCGCCGGGGCTTCACCAAGACCTACACTGTTGGCTGTGAGGAATGC
AACACCGAGGGACCTTGTCGGACTCGAATCGAGTCGCGGCCCCGAAGTGGTTCTGGATGTGACAACCGACACTCCTTACG

ACAGTGTTTCCCTGTTTATCCATCCCCTGCAAACTGCAGAGTGGCACTCATTGCTTGTGGACGGACCAGCTCCTCCAAGG
TGTCACAAAGGGACAAATAGGTAGGGGACGTTTGACGTCTCACCGTGAGTAACGAACACCTGCCTGGTCGAGGAGGTTCC

CTCTGAAAAGGGCTTCCAGTCCCGTCACCTTGCCTGCCTGCCTCGGGAGCCAGGGCTGTGCACCTGGCAGTCCCTGCGGT
GAGACTTTTCCCGAAGGTCAGGGCAGTGGAACGGACGGACGGAGCCCTCGGTCCCGACACGTGGACCGTCAGGGACGCCA

CCCAGATAGCCTGAATCCTGCCCGGAGTGGAAGCTGAAGCCTGCACAGTGTCCACCCTGTTCCCACTCCCATCTTTCTTC
GGGTCTATCGGACTTAGGACGGGCCTCACCTTCGACTTCGGACGTGTCACAGGTGGGACAAGGGTGAGGGTAGAAAGAAG

CGGACAATGAAATAAAGAGTTACCACCCAGCAAAAAAAAAAAAAAAAAAAAAAAAAAAAAAAAA
GCCTGTTACTTTATTTCTCAATGGTGGGTCGTTTTTTTTTTTTTTTTTTTTTTTTTTTTTTTTT

EP 0 189 784 B1

Fig. 2

```
GCA GGG GGG GGG GGG GGG GGG GGG GGG GGG GAC ATT TAT CCT CTA
GCG CTC AGG CCC TGC CGC CAT CGC CGC AGA TCC AGC GCC CAG AGA
                                        -20              -15
                        MET-ALA-PRO-PHE-GLU-PRO-LEU-ALA-SER-
GAC ACC AGA GAA CCC ACC ATG GCC CCC TTT GAG CCC CTG GCT TCT

          -10                      -5                      1
GLY-ILE-LEU-LEU-LEU-LEU-TRP-LEU-ILE-ALA-PRO-SER-ARG-ALA-CYS-
GGC ATC CTG TTG TTG CTG TGG CTG ATA GCC CCC AGC AGG GCC TGC


          5                   10                      15
THR-CYS-VAL-PRO-PRO-HIS-PRO-GLN-THR-ALA-PHE-CYS-ASN-SER-ASP-
ACC TGT GTC CCA CCC CAC CCA CAG ACG GCC TTC TGC AAT TCC GAC


          20                  25                      30
LEU-VAL-ILE-ARG-ALA-LYS-PHE-VAL-GLY-THR-PRO-GLU-VAL-ASN-GLN-
CTC GTC ATC AGG GCC AAG TTC GTG GGG ACA CCA GAA GTC AAC CAG


          35                  40                      45
THR-THR-LEU-TYR-GLN-ARG-TYR-GLU-ILE-LYS-MET-THR-LYS-MET-TYR-
ACC ACC TTA TAC CAG CGT TAT GAG ATC AAG ATG ACC AAG ATG TAT


          50                  55                      60
LYS-GLY-PHE-GLN-ALA-LEU-GLY-ASP-ALA-ALA-ASP-ILE-ARG-PHE-VAL-
AAA GGG TTC CAA GCC TTA GGG GAT GCC GCT GAC ATC CGG TTC GTC


          65                  70                      75
TYR-THR-PRO-ALA-MET-GLU-SER-VAL-CYS-GLY-TYR-PHE-HIS-ARG-SER-
TAC ACC CCC GCC ATG GAG AGT GTC TGC GGA TAC TTC CAC AGG TCC


          80                  85                      90
HIS-ASN-ARG-SER-GLU-GLU-PHE-LEU-ILE-ALA-GLY-LYS-LEU-GLN-ASP-
CAC AAC CGC AGC GAG GAG TTT CTC ATT GCT GGA AAA CTG CAG GAT
```

Fig. 2 cont.

```
                95                        100                       105
GLY-LEU-LEU-HIS-ILE-THR-THR-CYS-SER-PHE-VAL-ALA-PRO-TRP-ASN-
GGA CTC TTG CAC ATC ACT ACC TGC AGT TTT GTG GCT CCC TGG AAC

                 110                      115                       120
SER-LEU-SER-LEU-ALA-GLN-ARG-ARG-GLY-PHE-THR-LYS-THR-TYR-THR-
AGC CTG AGC TTA GCT CAG CGC CGG GGC TTC ACC AAG ACC TAC ACT

                125                       130                       135
VAL-GLY-CYS-GLU-GLU-CYS-THR-VAL-PHE-PRO-CYS-LEU-SER-ILE-PRO-
GTT GGC TGT GAG GAA TGC ACA GTG TTT CCC TGT TTA TCC ATC CCC

                140                       145                       150
CYS-LYS-LEU-GLN-SER-GLY-THR-HIS-CYS-LEU-TRP-THR-ASP-GLN-LEU-
TGC AAA CTG CAG AGT GGC ACT CAT TGC TTG TGG ACG GAC CAG CTC

                155                       160                       165
LEU-GLN-GLY-SER-GLU-LYS-GLY-PHE-GLN-SER-ARG-HIS-LEU-ALA-CYS-
CTC CAA GGC TCT GAA AAG GGC TTC CAG TCC CGT CAC CTT GCC TGC

                170                       175                       180
LEU-PRO-ARG-GLU-PRO-GLY-LEU-CYS-THR-TRP-GLN-SER-LEU-ARG-SER-
CTG CCT CGG GAG CCA GGG CTG TGC ACC TGG CAG TCC CTG CGG TCC


GLN-ILE-ALA-***
CAG ATA GCC TGA ATC CTG CCC GGA GTG GAA GCT GAA GCC TGC ACA

...
GTG TCC ACC CTG TTC CCA CTC CCA TCT TTC TTC CGG ACA ATG AAA

TAA AGA GTT ACC ACC CAG CAA AAA AGA AAA AAA AGA AAA AAA AAA
```

Fig. 3

PstI

GGG
CCC

← XhoII

← NcoI

← HincII

← AccI
← NcoI

← PstI
← PstI

← PstI

← AvaI

TTT
AAA

RsaI

4

**EP 0 189 784 B1**

Fig. 4         TIMP DNA SEQUENCES

```
CYS-THR-CYS-VAL-PRO-PRO-HIS-PRO-GLN-THR-ALA-PHE-CYS-ASN-SER-ASP-
TGY ACN TGY GTN CCN CCN CAY CCN CAR ACN GCN TTY TGY AAY OZE GAY

LEU-VAL-ILE-ARG-ALA-LYS-PHE-VAL-GLY-THR-PRO-GLU-VAL-ASN-GLN-THR-
YTB GTN ATL SGD GCN AAR TTY GTN GGN ACN CCN GAR GTN AAY CAR ACN

THR-LEU-TYR-GLN-ARG-TYR-GLU-ILE-LYS-MET-THR-LYS-MET-TYR-LYS-GLY-
ACN YTB TAY CAR SGD TAY GAR ATL AAR ATG ACN AAR ATG TAY AAR GGN

PHE-GLN-ALA-LEU-GLY-ASP-ALA-ALA-ASP-ILE-ARG-PHE-VAL-TYR-THR-PRO-
TTY CAR GCN YTB GGN GAY GCN GCN GAY ATL SGD TTY GTN TAY ACN CCN

ALA-MET-GLU-SER-VAL-CYS-GLY-TYR-PHE-HIS-ARG-SER-HIS-ASN-ARG-SER-
GCN ATG GAR OZE GTN TGY GGN TAY TTY CAY SGD OZE CAY AAY SGD OZE

GLU-GLU-PHE-LEU-ILE-ALA-GLY-LYS-LEU-GLN-ASP-GLY-LEU-LEU-HIS-ILE-
GAR GAR TTY YTB ATL GCN GGN AAR YTB CAR GAY GGN YTB YTB CAY ATL

THR-THR-CYS-SER-PHE-VAL-ALA-PRO-TRP-ASN-SER-LEU-SER-LEU-ALA-GLN-
ACN ACN TGY OZE TTY GTN GCN CCN TGG AAY OZE YTB OZE YTB GCN CAR

ARG-ARG-GLY-PHE-THR-LYS-THR-TYR-THR-VAL-GLY-CYS-GLU-GLU-CYS-THR-
SGD SGD GGN TTY ACN AAR ACN TAY ACN GTN GGN TGY GAR GAR TGY ACN

VAL-PHE-PRO-CYS-LEU-SER-ILE-PRO-CYS-LYS-LEU-GLN-SER-GLY-THR-HIS-
GTN TTY CCN TGY YTB OZE ATL CCN TGY AAR YTB CAR OZE GGN ACN CAY

CYS-LEU-TRP-THR-ASP-GLN-LEU-LEU-GLN-GLY-SER-GLU-LYS-GLY-PHE-GLN-
TGY YTB TGG ACN GAY CAR YTB YTB CAR GGN OZE GAR AAR GGN TTY CAR

SER-ARG-HIS-LEU-ALA-CYS-LEU-PRO-ARG-GLU-PRO-GLY-LEU-CYS-THR-TRP-
OZE SGD CAY YTB GCN TGY YTB CCN SGD GAR CCN GGN YTB TGY ACN TGG

GLN-SER-LEU-ARG-SER-GLN-ILE-ALA-
CAR OZE YTB SGD OZE CAR ATL GCN
```

KEY

A=A,   C=C,   G=G,   T=T,   Y=C or T,   R = A or G,

L = A or C or T,   N = A or C or T or G,

S = A or C,   Q = A or T,

```
IF S = A THEN D = R
IF S = C THEN D = N
IF Y = T THEN B = R
IF Y = C THEN B = N
IF Q = A THEN Z = G AND E = Y
IF Q = T THEN Z = C AND E = N
```

5

FIG. 5    TIMP AMINO TERMINAL SEQUENCE


CYS THR CYS VAL PRO PRO HIS PRO GLN THR
ALA PHE CYS ASN SER ASP LEU VAL ILE ARG
ALA LYS PHE VAL GLY THR PRO GLU VAL ---
GLU THR THR LEU TYR GLN ARG TYR GLU ILE
LYS MET --- LYS MET TYP LYS GLY PHE

Fig. 6

pAYE 4

Not drawn to scale

Fig. 7

pAYE 4 (34)

pAYE 4 (34)

Not drawn to scale

Fig. 8

YEAST PYRUVATE KINASE PROMOTER (Modified to introduce HindIII site)

AvaII

```
GGTCCCCTTTCAAAGTTATTCTCTACTCTTTTTCATA
CCAGGGGAAAGTTTCAATAAGAGATGAGAAAAAGTAT


TTCATTCTTTTTCATCCTTTGGTTTTTTATTCTTAAC
AAGTAAGAAAAAGTAGGAAACCAAAAAATAAGAATTG


TTGTTTATTATTCTCTCTTGTTTCTATTTACAAGCTT
AACAAATAATAAGAGAGAACAAAGATAAATGTTCGAA
                                HindIII
```

Fig.9

HindIII

pAYE 4 (34)

EcoR1

AvaII                    HindIII

AvaII

synthetic oligonucleotide

EcoR1

HindIII

Cut with EcoR1 and Xba1
Isolate 470bp fragment

Recut with AvaII
Isolate 330bp fragment

Isolate large
   HindIII-EcoR1
   fragment of
   pBR322

pAYE 85

HindIII

100bp

AvaII

330bp

EcoR1

10

Fig.10

pAYE 85

HindIII

AvaII

EcoR1

pAYE 4 (34)

EcoR1

AvaII

EcoR1

HindIII

Cut with HindIII and EcoR1
Isolate 440bp fragment

Cut with HindIII and EcoR1
Isolate 3kb fragment

p JDB 207 with ADHI terminator
cut with HindIII

LIGATE

EcoR1

EcoR1

HindIII

pAYE 86

EcoR1

AvaII

Pst1

HindIII

BamH1

Fig.11

EcoR1

EcoR1

HindIII

pAYE 86

EcoR1

AvaII

HindIII

Pstl

BamH1

Partial digestion with HindIII
DNA polymerase filling of sticky ends

LIGATE

EcoR1

EcoR1

HindIII
(removed in construction
of pAYE 89)

pAYE 89

Pstl

EcoR1 P.K. Promoter

AvaII

HindIII
Unique insertion site

BamH1